# EUROPÄISCHE PATENTSCHRIFT

(11) **EP 0 511 506 B1**
(45) Veröffentlichungstag und Bekanntmachung des Hinweises auf die Patenterteilung: **09.10.1996**
(21) Anmeldenummer: 92105570.3
(22) Anmeldetag: 01.04.1992
(51) Int. Cl.: A61B 17/225

(54) **Vorrichtung zur Fokalbereichsortung für die Lithotripsie**
Device for location of the focal region in lithotripsy
Dispositif pour localiser la zone focale en lithotripsie

(30) Priorität: 26.04.1991 DE 4113697
(43) Veröffentlichungstag der Anmeldung: 04.11.1992
(73) Patentinhaber: DORNIER MEDIZINTECHNIK GMBH, D-82101 Germering (DE)
(72) Erfinder: Treiber, Jobst, Dr., W-8000 München 90 (DE)
(74) Vertreter: Landsmann, Ralf, Dipl.-Ing.

(56) Entgegenhaltungen:
- EP-A- 0 257 199
- EP-A- 0 338 618
- EP-A- 0 367 116
- EP-A- 0 377 901
- DE-A- 3 517 934
- DE-A- 3 739 230
- US-A- 4 580 894

## Beschreibung

Die Erfindung betrifft eine Vorrichtung zur Ortung des Fokalbereichs der in einer Stoßwellenquelle erzeugten Stoßwellen relativ zu einem im Körper eines Lebewesens befindlichen Konkrement.

Der ESWL-Fokalbereich (ESWL = extrakorporale Stoßwellen-Lithotripsie) kann, besonders bei anatomisch ungünstigen Verhältnissen, erheblich vom geometrisch berechneten Fokus abweichen. Eine exakte Positionierung des zu therapierenden Konkrements ist in solchen Fällen nicht möglich, die ESWL-Effektivität ist entsprechend gering. Eine direkte Abbildung der Stoßwellenausbreitung/-Fokussierung im Körper ist mit den in der Medizin bekannten Bildgebungsverfahren nicht durchführbar.

In der **EP 367 116** wird der Fokalbereich indirekt durch die von den Stoßwellen induzierten Bewegungsvorgänge im Körper geortet. Bei dem dabei angewendeten Ultraschall-Dopplerverfahren kann allerdings nur die Radialgeschwindigkeit bezüglich des Ultraschallscanners gemessen werden, von dem die Ultraschallwellen ausgehen und deren Echos empfangen werden.

Der Erfindung liegt die Aufgabe zugrunde, eine Vorrichtung zu schaffen, mit der der Fokalbereich der Stoßwellenquelle zuverlässig geortet und das Konkrement sicher im Fokalbereich positioniert werden kann.

Diese Aufgabe wird erfindungsgemäß mit einer Vorrichtung mit den kennzeichnenden Merkmalen des Anspruch 1 gelöst. Ausgestaltungen der Erfindung sind Gegenstände von Unteransprüchen.

Mit der erfindungsgemäßen Vorrichtung wird die Stoßwellenausbreitung/-Fokussierung innerhalb des Körpers indirekt abgebildet durch Erfassung der durch die Stoßwellen induzierten Bewegungsvorgänge.
Bewegungen können induziert werden im Steinmaterial, in der das Konkrement umgebenden Flüssigkeit oder im Körpergewebe. Eine weitere Möglichkeit ist die Bewegung von im Stoßwellenfeld erzeugten Kavitationsblasen. Da diese Bewegungsvorgänge im Fokalbereich die stärkste Ausprägung finden, ist eine Ortung dieser Region möglich.
Die Erfassung der induzierten Bewegungsvorgänge im Innern des Patientenkörpers erfolgt nichtinvasiv durch Auswertung des für die ESWL-Steinortung verwendeten Ultraschall-B-Bildes, im folgenden auch B-Bild genannt. Die Bewegungen werden in einer Vorrichtung zur Detektion der durch die Stoßwellen induzierten Bewegungsvorgängen durch Segmentierung und Korrelation aufeinanderfolgender B-Bilder bestimmt. Dabei ist der Vergleich nicht auf direkte Folgebilder Bᵢ, Bᵢ₊₁ beschränkt, es können vorteilhaft auch Bilder Bᵢ, Bᵢ₊ₖ mit k > 1 verglichen werden. Die ermittelten Geschwindigkeitswerte werden zur Erzeugung eines farbkodierten Geschwindigkeitsbildes verwendet und können dem Ultraschall-B-Bild überlagert werden.
Bei einem solchen Geschwindigkeitsbild ist jeder Kombination von Betrag/Geschwindigkeit eine Farb/Intensitätskombination zugeordnet.
Die dem B-Bild inhärenten Abbildungsfehler durch Schallbrechung und Schallbeugung sind für die Fokalbereichortung hier ohne Einfluß, da für die ESWL-Positionierung die relative Position von Fokalbereich zum Konkrement entscheidend ist.

Die Erfindung wird anhand von Figuren näher erläutert.

Es zeigen:
- Fig. 1: eine schematische Darstellung der von den Stoßwellen induzierten Bewegungsvorgänge im Körper
- Fig. 2: das Blockschaltbild einer erfindungsgemäßen Vorrichtung
- Fig. 3: das Blockschaltbild der Vorrichtung BD zur Detektion der von den Stoßwellen induzierten Bewegungsvorgängen

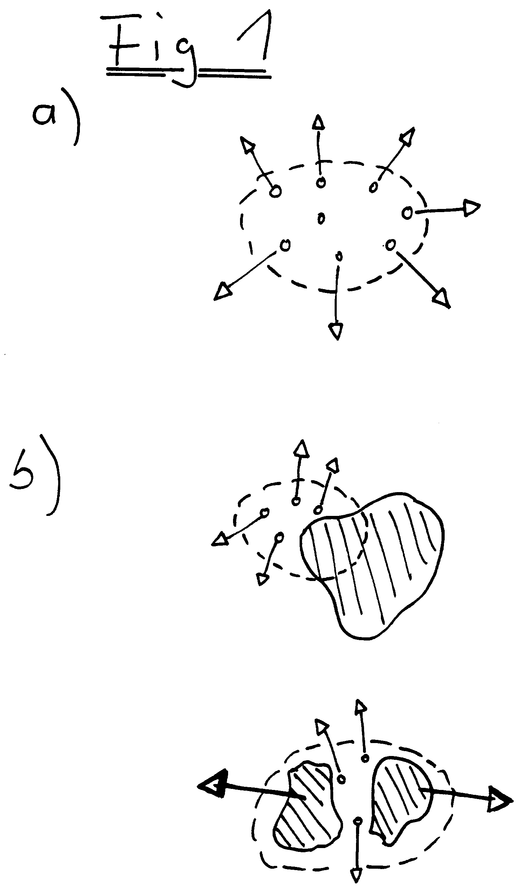

Fig. 1 zeigt schematisch die von den Stoßwellen induzierten Bewegungsvorgänge, die zur Ortung des Fokalbereichs dienen können. Der Fokalbereich ist jeweils durch eine gestrichelte Markierung umrandet und die Bewegungsrichtungen durch Pfeile dargestellt.
In Fig. 1a ist die Dynamik von Kavitationsblasen, die in einer Flüssigkeit innerhalb des Fokalbereichs der Stoßwellen entstehen, dargestellt. Die Kavitationsblasen wirken als bewegtes Ultraschallkontrastmedium und sind deshalb im Ultraschall-B-Bild sichtbar.
In Fig. 1b ist die Dynamik von Steinmaterial innerhalb des Fokalbereichs dargestellt. Große Steine (obere Abbildung) verhalten sich im wesentlichen immobil, so daß vor allem die in der umgebenden Flüssigkeit entstehenden Kavitationsblasen zur Ortung des Fokalbereichs dienen.

In Fig. 1b, untere Abbildung, ist die Dynamik von Steinfragmenten dargestellt. Hier sind sowohl die Bewegung der Steinfragmente als auch die in der umgebenden Flüssigkeit entstehenden Kavitationsblasen zur Ortung des Fokalbereichs geeignet.

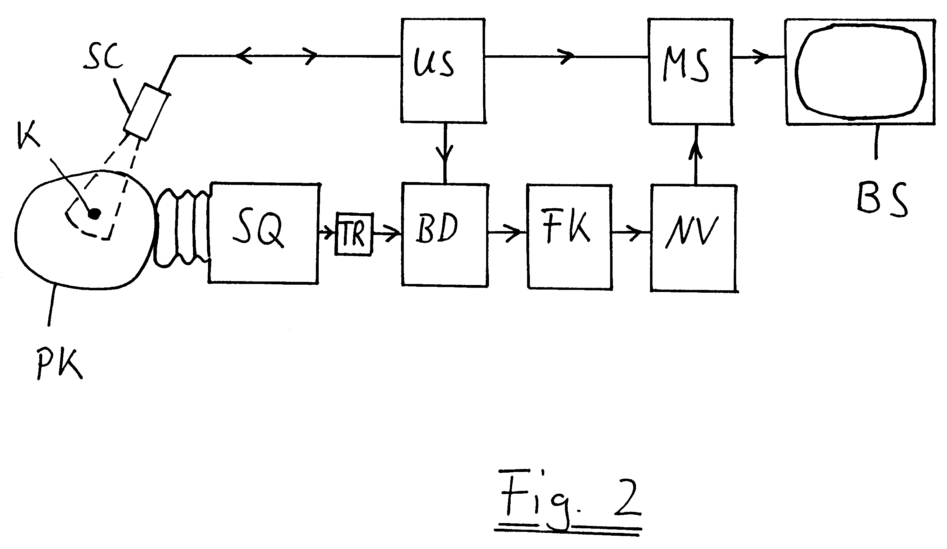

Fig. 2 zeigt das Blockschaltbild einer erfindungsgemäßen Vorrichtung. Im Patientenkörper **PK** befindet sich das zu therapierende Konkrement **K**. In der Stoßwellenquelle **SQ** werden Ultraschallstoßwellen erzeugt, fokussiert und auf das Konkrement **K** ausgerichtet. Ein Ultraschallscanner **SC** sendet Ultraschallwellen und empfängt deren Echos, die in der Ultraschallvorrichtung **US** zu einem Ultraschall-B-Bild des betrachteten Körperbereichs weiterverarbeitet werden. Das B-Bild wird in einer Vorrichtung **BD** zur Detektion der durch die Stoßwellen induzierten Bewegungsvorgänge weitergeleitet. Die Detektion erfolgt dort durch Segmentierung und Korrelation zweier aufeinanderfolgender, also zu verschiedenen Zeiten aufgenommener B-Bilder Bᵢ, Bᵢ₊ₖ mit k=> 1. Durch eine Triggereinheit **TR** wird die Bewegungsdetektion beschränkt auf ein Zeitfenster nach Auslösung der Stoßwellen in der Stoßwellenquelle **SQ**. In der Vorrichtung **FK** werden die berechneten Geschwindigkeitswerte farbkodiert, so daß der in den B-Bildern abgebildete Bereich des Patientenkörpers **PK** in ein Geschwindigkeitsbild gewandelt wird. Anschließend erfolgt in der Vorrichtung **NV** eine Nachverarbeitung des farbkodierten Bildes (z.B. Glättung, Interpolation).
In einer Mischstufe **MS** wird das farbkodierte Geschwindigkeitsbild mit dem Ultraschall-B-Bild überlagert und auf einem Bildschirm **BS** sichtbar gemacht.

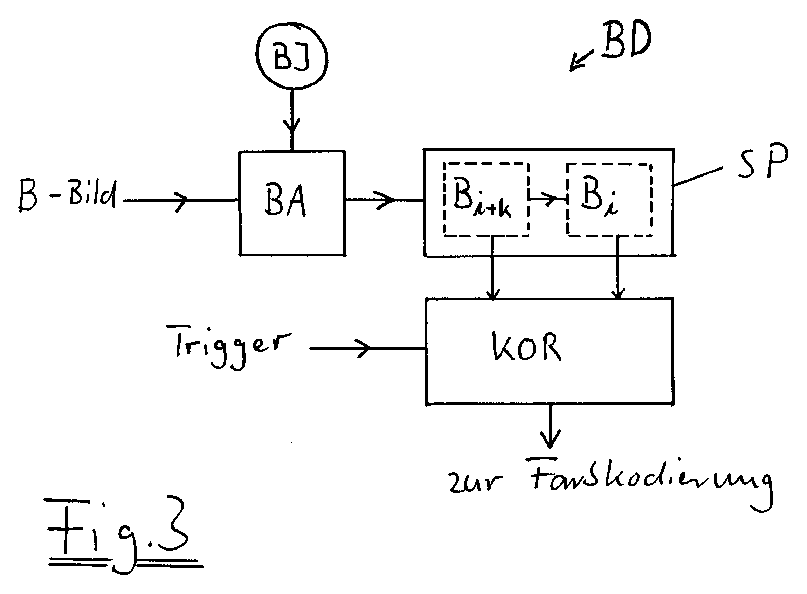

Fig. 3 zeigt das Blockschaltbild einer Ausführung der Vorrichtung **BD** zur Detektion der von dem Stoßwellen induzierten Bewegungsvorgängen. Im Bildspeicher **SP**, der z.B. als Schiebespeicher ausgelegt ist, werden zwei aufeinanderfolgende B-Bilder Bᵢ,Bᵢ₊ₖ, mit k >= 1, gespeichert. Mit der Vorrichtung **BA** kann der angenommene Fokalbereich, im folgenden auch ROI (Region-Of-Interest) genannt, innerhalb des B-Bildes über ein Benutzerinterface **BI** festgelegt werden, so daß nur innerhalb dieses ROI Bewegungsvorgänge detektiert werden.
Im Korrelator **KOR** werden die beiden ROI nach einem, z.B. quadratischen Raster in Bildsegmente geeigneter Größe aufgeteilt. Ein Segment umfaßt dabei in der Regel mehrere Bildpunkte (pixel). Anschließend werden die Korrelationskoeffizienten zwischen jeweils einem Segment im ersten Bild Bᵢ und der Bildumgebung des entsprechenden Segments im Folgebild Bᵢ₊ₖ berechnet. Grundlage für die Berechnung sind die Graustufenwerte der einzelnen Bildpunkte. Der Differenzvektor zwischen dem Segment im ersten Bild Bᵢ und dem Bereich maximaler Korrelation im Folgebild wird zur Berechnung von Betrag und Geschwindigkeit des Bewegungsvektors verwendet. Aus dem Korrelationsprozeß hervorgehende, jedoch physikalisch unsinnige Schätzwerte können erkannt und herausgefiltert werden.
Die Gesamtheit der ermittelten zweidimensionalen Geschwindigkeitsvektoren werden bei der anschließenden Farbkodierung in ein Geschwindigkeitsbild gewandelt. Jedem Bildsegment ist dabei eine Farbe/Intensitäts-Kombination für Richtung und Betrag zugeordnet.
Durch die Triggereinheit **TR** wird die Bewegungsdetektion auf stoßwellenrelevante Zeiten beschränkt.
Gegenüber der Detektion der induzierten Bewegungsvorgänge nach dem Dopplerverfahren besitzt die erfindungsgemäße Vorrichtung den Vorteil der Erfassung von zweidimensionalen Geschwindigkeitskomponenten. Somit kann sowohl das B-Bild eines In-Line-Scanners wie auch eines Off-Axis-Scanners verwendet werden ohne Einbußen in der Detektion von Bewegungsvorgängen in einer etwaigen Vorzugsrichtung.

## Patentansprüche

1. Vorrichtung zur Ortung des Fokalbereiches der in einer Stoßwellenquelle (SQ) erzeugen Stoßwellen relativ zu einem im Körper (PK) eines Lebenwesens befindlichen Konkrements (K), mit
- Ultraschalleinrichtungen (SC, US) zur Erzeugung von Ultraschall-B-Bildem eines Bildbereichs innerhalb des Körpers (PK),
- einer Vorrichtung (BD) zur Detektion von durch die Stoßwellen im Körper (PK) induzierten Bewegungsvorgängen,
- einer Triggereinheit (TR) zur Beschränkung der Bewegungsdetektion auf ein festgelegtes Zeitfenster nach Auslösung der Stoßwellen,
- einer Vorrichtung (FK) zur Erzeugung eines farbkodierten Geschwindigkeitsbildes des Bildbereichs und
- einer Vorrichtung (MS) zur Überlagerung des farbkodierten Geschwindigkeitsbildes mit einem Ultraschall-B-Bild,
**dadurch gekennzeichnet,** daß die Vorrichtung (BD) zur Detektion der Bewegungsvorgange einen Bildspeicher (SP) zur Abspeicherung zweier Ultraschall-B-Bilder (Bᵢ, Bᵢ₊ₖ) und eine Korrelationsvorrichtung (KOR) enthält, in der die beiden gespeicherten Ultraschall-B-Bilder (Bᵢ, Bᵢ₊ₖ) in Bildsegmente unterteilt werden und Bildsegmente des einen Ultraschall-B-Bildes (Bᵢ) mit der Umgebung des entsprechenden Segments im anderen Ultraschall-B-Bild (Bᵢ₊ₖ) korreliert werden.

2. Vorrichtung nach Anspruch 1, **dadurch gekennzeichnet,** daß die Vorrichtung (**BD**) zur Detektion der Bewegungsvorgänge eine zusätzliche Vorrichtung (**BA**) zur Festlegung einer Region-Of-Interest (angenommener Fokalbereich) innerhalb eines Ultraschall-B-Bildes (Bᵢ,Bᵢ₊ₖ) enthält, so daß nur innerhalb der Region-Of-Interest Bewegungsvorgänge detektiert werden.

3. Vorrichtung nach einem der vorangehenden Ansprüche, **dadurch gekennzeichnet**, daß eine Vorrichtung (**NV**) zur Nachverarbeitung des farbkodierten Geschwindigkeitsbildes (z.B. zur Glättung und Interpolation) vorhanden ist.

## Claims

1. Device for determining the focal range of shockwaves generated in a shockwave source (SQ) relative to a concrement (K) located in the body (PK) of a living organism, comprising
- ultrasound devices (SC, US) for generating ultrasound-B-images of an image area within the body (PK);
- a device (BD) for detecting movement processes induced in the body (PK) by shockwaves;
- a trigger unit (TR) to restrict movement detection to a specified time window after triggering of the shockwaves;
- a device (FK) for generating a colour-coded velocity image of the image range; and
- a device (MS) for superpositioning the colour-coded velocity image with an ultrasound-B-image;
**characterized in that** the device (BD) for the detection of movement processes comprises an image store (SP) for storing two ultrasound-B-images (Bᵢ, Bᵢ₊ₖ) and a correlation device (KOR) wherein the two stored ultrasound-B-images (Bᵢ, Bᵢ₊ₖ) are divided into image segments and the image segments of the one ultrasound-B-image (Bᵢ) are correlated with the surrounding of the respective segment in the other ultrasound-B-image (Bᵢ₊ₖ).

2. Device according to Claim 1, **characterized in that** the device (BD) for detection of movement processes incorporates an additional device (BA) for determining a region of interest (assumed focal range) within an ultrasound-B-image (Bᵢ, Bᵢ₊ₖ), so that movement processes are only detected within the region of interest.

3. Device according to one of the above claims, **characterized in that** a device (NV) is provided for subsequent processing of colour-coded velocity images (for example by smoothing and interpolation).

## Revendications

1. Dispositif pour la localisation de la zone focale des ondes de choc produites dans une source d'ondes de choc (SQ) par rapport à une concrétion (K) se trouvant dans le corps (PK) d'un être vivant, comprenant
- des dispositifs à ultrasons (SC, US) pour produire des images ultrasons B d'une zone d'image à l'intérieur du corps (PK),
- un dispositif (BD) pour la détection des mouvements induits par les ondes de choc dans le corps (PK),
- un dispositif déclencheur (TR) pour la limitation de la détection de mouvements sur une fenêtre de temps fixe après déclenchement des ondes de choc,
- un dispositif (FK) pour produire une image de vitesse à codage couleur de la zone d'image, et
- un dispositif (MS) pour la superposition de l'image de vitesse à codage en couleur avec une image ultrasons B,
caractérisé par le fait que le dispositif (BD) pour la détection des mouvements comprend une mémoire d'images (SP) pour la mémorisation de deux images ultrasons B (Bᵢ, Bᵢ₊ₖ) et un dispositif de corrélation (KOR) dans lequel les deux images ultrasons B mémorisées (Bᵢ, Bᵢ₊ₖ) sont subdivisées en segments d'image et les segments d'image de l'une des images ultrasons B (Bᵢ₊ₖ) sont mises en corrélation avec l'environnement du segment correspondant dans l'autre image ultrasons B (Bᵢ₊ₖ).

2. Dispositif suivant la revendication 1, caractérisé par le fait que le dispositif (BD) pour la détection des mouvements comprend un dispositif supplémentaire (BA) pour la détermination d'une zone d'intérêt (zone focale supposée) à l'intérieur d'une image ultrasons B (Bᵢ, Bᵢ₊ₖ) de manière que des mouvements ne soient détectés qu'à l'intérieur de la zone d'intérêt.

3. Dispositif suivant l'une des revendications précédentes, caractérisé par le fait qu'il est prévu un dispositif (NV) pour le traitement ultérieur de l'image de vitesse à codage couleur (par exemple à des fins de lissage et d'interpolation).
